# EUROPEAN PATENT SPECIFICATION

(11) **EP 3 218 764 B1**
(45) Date of publication and mention of the grant of the patent: **06.03.2024**
(21) Application number: 15794597.3
(22) Date of filing: 16.11.2015
(51) Int. Cl.: G02C 11/08, G02C 9/00, A61F 9/02

(54) **CLOSED GLASSES WITH INTEGRATED OPTICAL LENSES**
GESCHLOSSENE BRILLE MIT INTEGRIERTEN OPTISCHEN LINSEN
LUNETTES FERMÉES AVEC LENTILLES OPTIQUES INTÉGRÉES

(30) Priority: 14.11.2014 BE 201405064
(43) Date of publication of application: 20.09.2017
(73) Proprietor: Xvision, 3191 Hever (BE)
(72) Inventor: VAN DEN BOGAERT, Jeroen, 2500 Lier (BE); VAN ROY, Wim, 3191 Hever (BE); VAN ROY, Jan, 3191 Hever (BE)
(74) Representative: Winger
(86) International application number: PCT/EP2015/076729
(87) International publication number: WO 2016/075338

(56) References cited:
- WO-A1-2014/154370
- DE-U1-202013 104 927
- DE-U1-202014 002 709
- FR-A1- 2 688 322
- FR-A1- 2 688 322
- US-A- 2 799 862
- US-A- 2 799 862
- US-A- 3 395 406
- US-A- 5 371 555
- US-A- 5 790 230
- US-A- 5 790 230
- US-A1- 2002 029 408
- US-A1- 2014 063 438
- US-A1- 2014 063 438
- US-B1- 6 637 877
- US-B1- 6 637 877
- US-B1- 7 866 812

## Description

### Scope of the invention

In general this invention is related to the field of winter sports for people with visual impairments. More specifically it relates to closed glasses, suitable for winter sports, for example ski goggles, with integrated optical lenses.

### Background of the invention

The problems experienced by people with positive or negative dioptre during winter sports today are limiting the participation to and the pleasure of winter sports. Although a number of solutions exist to correct sight for people with visual impairments these solutions are in general accompanied by a number of additional problems.

A first solution to correct for visual impairments during winter is to wear contact lenses under conventional goggles. However, a large group of people feel uncomfortable with the use of contact lenses, which suffer for example from dryness of the eyes or suffering from allergies when using them. For this group, the use of contact lenses is not an option.

A second solution that is currently during winter is used by people with visual impairment is the use of Over The Glasses (OTG) ski goggles. These goggles are wider, leave more space for the frame of the glasses and are further away from the face than the standard ski goggles. This way glasses with the optical correction lenses can be worn underneath the ski goggles. One of the big disadvantages of this solution is condensation on both sides of the correction lens limiting the sight significantly. Moreover, this solution is faced with an additional problem. Modern ski goggles are today made with double visors. This means that the light will be filtered by each of the material transitions before it gets to the retina. At each of these transitions light is lost and therefore less light is getting on the eye. A further disadvantage of carrying the own glasses under the OTG ski goggle is the risk of injury in case of a possible fall. The frame of one's own spectacles, as well as the glass could cause injury, as they are not designed to absorb the energy of a fall.

A third solution actually used, is a clip inserted in the inside of the actual ski goggle. In this clip an optician can mount corrective lenses but as the distance to the eye is not optimal the dioptres possible are limited from a dioptre of +3 to a dioptre of -3. Furthermore the solution with the clips will also have problems with condensation on both sides of the corrective lenses, the light will be filtered three times and the user will suffer from vibrations if the clip is not attached extremely rigid.

Examples for prior art solutions can be found in US3395406, DE02013104927U, US5371555 and US7866812.

Consequently, there is room for a more efficient system for the placement of optical lenses in goggles.

### Summary of the invention

It is an object of embodiments of the present invention to provide comfortable closed glasses for people with visual impairments during winter sports, more specifically but not limited to alpine skiing or snowboarding.

It is an advantage of at least some embodiments of the present invention that problems with condensation can be reduced or even avoided.

It is an advantage of at least some embodiments of the present invention that the obtained closed glasses allow correction for smaller as well as larger dioptres (covering dioptres up to -10) and other deviations as astigmatism. This means that this solution offers a solution to a large group of people, possibly larger than the group of people that can make use of state of the art solutions.

It is an advantage of at least some embodiments of the present invention that the resulting closed glasses reduce the risk of injury while skiing compared to using glasses and an OTG ski goggle.

It is an advantage of at least some embodiments of the present invention that the resulting closed glasses provide an integrated solution i.e. the embodiments provide a solution in one part making separate OTG ski goggles, own (sports) spectacles, clips, etc. unnecessary.

A device according to embodiments of the present invention can realize the above-mentioned object.

In a first aspect the present invention relates to a lens holder, e.g. a 3 dimensional lens holder, for mounting or positioning at least one optical lens for correction of visual impairment of a user in goggles suitable for winter sports. The 3D-lens holder comprises an outer edge allowing it to be mounted in a closely fitting manner to an outer lens of the goggles, e.g. on the inside of the outer lens (visor) of goggles for winter sports. The lens holder is adapted for allowing the mounting or positioning of at least one optical lens, e.g. a corrective lens, in a way that when the 3D lens holder is connected to the visor in a closely fitting manner, at least one optical lens is positioned outside of the minimal surface determined by the external edge of the lens holder. The minimal surface may be parallel with the outer lens of the goggles, e.g. visor. The lens holder is also adapted for creating at least one closed volume included between the contours of the lens holder, the at least on optical lens and the outer lens, e.g. visor, of the goggles for winter sports.

It is an advantage that the embodiments of the present invention allow that optical lenses can be integrated in the ski goggles by means of a lens holder.

It is an advantage of at least some embodiments of the present invention that these allow that the optical lenses can be integrated in the ski goggles so that condensation on the optical lenses can be reduced or even avoided.

It is an advantage of at least some embodiments of the present invention that these allow that the 3D lens holder, the optical lenses and the visor (outer lens) form at least one closed volume. This way a closed volume is created with air creating isolation between the inner - and the outer side of the ski goggles. This isolated volume is avoiding condensation on the side of the corrective lens within the closed volume and reduces the risk of condensation on the other side of the corrective lens.

It is an advantage of at least some embodiments of the present invention that these reduce the number of filtrations of the light to two compared to a solution with clips or glasses. This way the light has only been attenuated twice before reaching the eye.

A device according to embodiments of the present invention can realize the above-mentioned object.

In a first aspect the present invention relates to a lens holder, e.g. a 3 dimensional lens holder, for mounting or positioning at least one optical lens for correction of visual impairment of a user in goggles suitable for winter sports. The 3D-lens holder comprises an outer edge allowing it to be mounted in a closely fitting manner to an outer lens of the goggles, e.g. on the inside of the outer lens (visor) of goggles for winter sports. The lens holder is functioning as inner lens of the goggles and is adapted for allowing the mounting or positioning of at least one optical lens, e.g. a corrective lens, in a way that when the 3D lens holder is connected to the visor in a closely fitting manner by sealing attachment to the outer lens of the goggles, at least one optical lens is positioned outside of the minimal surface determined by the external edge of the lens holder. The minimal surface may be parallel with the outer lens of the goggles, e.g. visor. The lens holder is also adapted for creating at least one closed volume included between the contours of the lens holder, the at least on optical lens and the outer lens, e.g. visor, of the goggles for winter sports. Furthermore, the at least one optical lens comprises a first optical lens and a second optical lens, and the optical axis of the first optical lens and the second optical lens are intersecting at the side of the eyes at an angle smaller than 10°.

It is an advantage that the embodiments of the present invention allow that optical lenses can be integrated in the ski goggles by means of a lens holder.

It is an advantage of at least some embodiments of the present invention that these allow that the optical lenses can be integrated in the ski goggles so that condensation on the optical lenses can be reduced or even avoided.

It is an advantage of at least some embodiments of the present invention that these allow that the 3D lens holder, the optical lenses and the visor (outer lens) form at least one closed volume. This way a closed volume is created with air creating isolation between the inner - and the outer side of the ski goggles. This isolated volume is avoiding condensation on the side of the corrective lens within the closed volume and reduces the risk of condensation on the other side of the corrective lens.

It is an advantage of at least some embodiments of the present invention that these reduce the number of filtrations of the light to two compared to a solution with clips or glasses. This way the light has only been attenuated twice before reaching the eye.

In this case there is no additional attenuation caused by an additional lens (mounted in the regular glasses or the clips)
It is an advantage of at least some embodiments of the present invention that these enable a vertex distance between 0 and 20 mm between the eye and at least one optical lens when the 3D lens holder has been mounted together with the visor of the goggles for winter sports and is used by the user.

It is an advantage of at least some embodiments of the present invention that these enable that at least one optical lens is positioned at the optimal optical distance for the correction of the eyes.

It is an advantage of at least some embodiments of the present invention that these are also useful for higher dioptres (not only for dioptres up to -3). By the use of different corrective lenses embodiments of this present invention will allow the realization of ski goggles for dioptres up to -10 or other eye corrective needs (such as astigmatism). In realizations of the present invention the vertex distance can be situated between 0 and 20 mm but also between 5 and 20 or for example between 14 and 17 mm.

It is an advantage of at least some embodiments of the present invention that these enable the possibility that if two optical lenses are used the optical axes can be deviating between them. An angle smaller than 10° between the two axes is possible. The optical axes of the first and second optical lens (typically introduced as corrective lenses for both eyes) can make an angle smaller than 10°. More particularly, when the optical axes of the first and second optical lens may intersect at the side of the eyes, when the user is wearing the goggles, and the angle of intersection may be smaller than 10° e.g. smaller than 5°.

It is an advantage of at least some embodiments of the present invention that these allow that the corrective lenses are mounted in parallel or with a very small angle compared to the eyes creating the best possible image.

It is an advantage of at least some embodiments of the present invention that these enable the positioning of the corrective lenses under a very small angle creating more distance between the optical lenses and the eyes. This means that a better ventilation of the lenses is possible. The choice of the angle is also depending upon the desired position of the optical lenses. Optically the optimal position of the optical axis and the eye axis are the same. This means that the choice of the angle is a trade-off between the optimal ventilation and the optimal position of the optical lens. In actual embodiments of the present invention this angle may be situated between -10° and +10°, preferably between -5° and +5°.

It is an advantage of at least some embodiments of the present invention that these enable the positioning of the optical lens in order to obtain the best possible ventilation of the optical lens. The angle of the optical axis of the first lens and the optical axis of the second lens can be smaller than 5°.

It is an advantage of at least some embodiments of the present invention that these enable more ventilation space by positioning the optical lenses under a relative angle to each other. This additional ventilation space decreases the chances of condensation. The effect of an angle between the optical axes of the optical lenses is that he viewing direction is changing. This effect becomes bigger as the dioptre of the optical lenses gets bigger (bigger dioptres or other deviations). In embodiments of the present invention the axis between both optical axes have been made smaller than 10°, for instance smaller than 5°. This can be realized by using a lens holder deviating from the minimum surface using the edge of the lens holder and in parallel with the visor. The present invention is using a specific 3D design allowing to orient the optical lenses differently and closer to the eyes than what is possible by positioning optical lenses in the conventional inner lens from a ski goggle.

It is an advantage of at least some embodiments of the present invention that these enable that the design of the lens holder allow control of the position of the optical lenses with respect to:
- Vertex distance of 14 to 17 mm
- The angle between the optical axes of the optical lenses
- The angle between the optical axes and the eye axis (in actual embodiments of the present invention this angle can be in-between 0 and 5°)

This allows the use of optical lenses for higher dioptres than -1 to -3. In actual realizations of the present invention dioptres of -10 are possible.

At least one optical lens can be fixedly positioned in the opening of the 3D lens holder. It is an advantage of at least some embodiments of the present invention that these enable mechanical fixation of at least one optical lens in the 3D lens holder and that this optical lens cannot come loos under the influence of shocks.

The at least one optical lens can include a groove and the edge of the at least one opening of the 3D lens holder may be adapted to fit to the at least one optical lens groove to allow a fixed mechanical mounting.

The at least one optical lens can mechanically be fixed in at least one of the openings of the 3D lens holder en the adhesion can be improved by means of silicon rubber or glue or a combination of both.

The 3D lens holder and/or one or more optical lenses positioned in the 3D lens holder can be provided with an anti-condensation coating.

The present invention also relates to glasses suitable for winter sports comprising a 3D lens holder as describe above.

This 3D lens holder can be bonded to the visor (outer lens) of the ski goggle so that the visor, the optical lenses and the lens holder forms a closed space.

It is an advantage of at least some embodiments of the present invention that these enable the realization of a comfortable ski goggles with integrated optical lenses for people with visual impairment.

It is an advantage of at least some embodiments of the present invention that the formation of condensation on the optical lenses is avoided. Due to the presence of at least one closed volume there is an insulating layer of air present between the optical lens and the visor (outer lens). Therefore the optical lens is not in direct contact with the cold outside air. As a result the optical lens and the 3D lens holder are less cold than if in direct contact with the outside air. This ensures that humid air warmed by the body heat is less rapidly condensing against the optical lens. The boundaries of this closed space in the embodiments of the present invention are the visor, the 3D lens holder and the optical lenses which are kept together by a foam or other mechanical means.

Ventilation openings and/or a fan may be present and/or positioned so that at least one optical lens is located in the ventilation streams coming from the vents or the fan. It is an advantage of at least some embodiments of the present invention that these avoid the formation of condensation on the optical lenses.

It is an advantage of at least some embodiments of the present invention that these allow that the position of the 3D lens holder and the position of the vents and/or fans can be matched to each other to optimize the airflow over the optical lenses and to avoid condensation on them.

The surface of the goggles may be containing an anti-fog coating. It is an advantage of at least some embodiments of the present invention that these enable the avoidance of formation of condensation by the presence of an anti-fog coating, more specifically on the optical lenses.

The lens holder and the ski goggles may be mounted so as they form a single piece. The latter may result in the fact that the risk of injury when falling during winter sports is reduced.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

### Brief description of the drawings

The invention will now be described further, by way of example, with reference to the accompanying drawings, wherein like reference numerals refer to like elements in the various figures.
FIG. 1 illustrates a schematic top and front view of a 3D lens holder in accordance with embodiments of the present invention.
FIG. 2 illustrates a schematic back view of a 3D lens holder in accordance with embodiments of the present invention.
FIG. 3 illustrates an exploded view of ski goggles visor insert with a double optical lens and a 3D lens holder in accordance with embodiments of the present invention.
FIG. 4 illustrates a top view of ski goggles visor insert with a double optical lens and a 3D lens holder in accordance with embodiments of the present invention.
FIG. 5 illustrates a top view of a 3D lens holder in accordance with embodiments of the present invention.
FIG. 6 illustrates a top view of a 3D lens holder in accordance with embodiments of the present invention.

The figures are only schematic and are not limiting. For illustration purposes the dimensions of the parts can be over dimensioned and not in the same scale.

Reference numbers in the conclusions should not be interpreted to limit the scope of the protection. The same reference numbers in the different illustration are making a reference to the same or similar elements.

### Detailed description of illustrative embodiments

The present invention will be described with reference to particular embodiments and with reference to certain drawings but the invention is not limited thereto but only limited by the claims. The drawings described are only schematic and non-limiting. For illustration purposes the dimensions of the parts can be over dimensioned and not in the same scale. The dimensions and the relative dimensions may deviate from the actual practical embodiment of the invention.

Furthermore the terms first, second, third and the like in the description and in the claims are used for distinguishing between, similar elements and not necessarily for describing a sequence, either temporarily, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps, or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect inventive aspects lie in less than all features of a single foregoing disclosed embodiment. Thus, the claims following the detailed description are hereby expressly incorporated into this detailed description with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

It should be noted that the use of particular terminology when describing certain aspects of the invention should be taken to imply that the terminology is being redefined herein to be restricted to include any specific characteristics of the features or aspects of the invention with which that terminology is associated.

In the description provided herein, numerous specific details are set forth. However, it is understood that embodiments of the invention may be practiced without these specific details. In other instances, well-known methods, structures and techniques have not been shown in detail in order not to obscure an understanding of this description.

Where in the embodiments of the present invention, reference is made to a minimal surface defined by the outer edge, reference is made to the surface that would be defined by the surface of the inner lens of a conventional ski goggle. The minimal surface can thereby be defined as the surface that substantially corresponds with the surface that is formed by a soap bubble over the perimeter of the inner lens.

Where in the embodiments of the present invention reference is made to a "an optical lens", this refers to a lens trough which light beams can be made convergent or divergent. This is different from an outer lens of a ski goggle as this outer lens is only meant to filter out the sunlight. Typically ski goggles have an outer lens to filter the sunlight and 1 inner lens. Both the inner and outer lens can be coated (colour, polychromatic, polarised) to increase visibility on the slopes. Due to the isolated space between the outer lens and the inner lens, the formation of condensation on the inner lens is decreased. In the embodiments of the present invention the inner lens is replaced by the 3D lens holder and optical lenses.

Where in the embodiments of the present invention, reference is made to the "inside of an inner lens", the side of the inner lens closest to the face is meant. The term lens can make reference to as well an optical lens as a sunlight filtering lens.

In embodiments of the present invention the optical lenses can be made amongst others out of glass or polycarbonate. Furthermore within embodiments of the present invention "standard" optical lenses can be used. These lenses can be adapted to the specific optical characteristics as pupil distance, astigmatism and /or dioptre on the standard equipment of opticians. The optical lens may be coated with different materials to reduce glare and the formation of condensation.

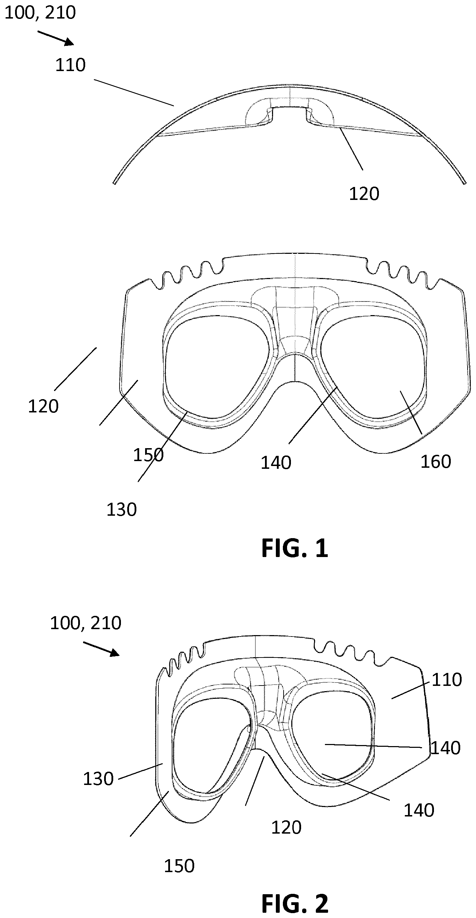

In a first aspect the present invention relates to a 3D lens holder for the mounting or positioning of at least one optical lens for the correction of visual impaired people in goggles suitable for winter sports, such as ski goggles. However the present invention is not limited to ski goggles but it can concern closed glasses for sports, whereby the control of solar light can be realized. Examples of such sports are mountaineering, Nordic walking, walking in the snow, snow scooting,... In embodiments of the present invention, the outer edge of the lens holder can be mechanically fixed in a closely fitting manner, e.g. sealed, to the outer lens of the goggles for winter sports, e.g. visor of the ski goggles. The lens holder is also suitable for the fixation or positioning of at least one optical lens. When the lens holder is mechanically fixed to the outer lens, at least one optical lens is positioned outside of the minimum surface defined by the outer edge of the 3D lens holder. Furthermore at least 1 closed space is defined by the outer lens, the at least one optical lens and the lens holder in the goggles. The lens holder 100, an example thereof being schematically illustrated in FIG. 1, functions in the present invention as the inner lens 210 of the goggles for winter sports, e.g. ski goggles. The lens holder has an outer edge that can be connected closely to the outer lens of the ski goggle. Typically the outer edge 110 of the lens holder has the same flexion as the outer lens (visor). The 3 D lens holder also comprises a part 120 in which one or more optical lenses 130, 140 can be integrated. To that extend, a first opening 150 and a second opening 160 are provided in the region 120 of the lens holder 100. When the 3D lens holder is mounted in the goggles and when the optical lenses are mounted or positioned in the lens holder, the optical lenses 130, 140 will typically be closer to the eyes than the inner lens of a conventional ski goggle. The lens holder has been designed that the optical lenses are positioned outside the minimal surface defined by the contour of the external edge of the lens holder. Furthermore the lens holder, the optical lenses 130, 140 and the outer lens 310 of the goggles create a closed volume.
The lens holder 100 can be made from a diversity of materials such as one or a combination of different sorts of plastics such as, for example, polycarbonate, polymethyl methacrylate, polyethylene terephthalate, polypropylene, polyoxymethylene or polyacetat.

FIG. 1 shows an example of an embodiment of the present invention. Both a top and a front view are shown. The external edge 110 of the lens holder is designed in such fashion that it has the same flexion as the outer lens 210 of the goggles and such that it can be attached closely to the outer lens of the goggles. In embodiments of the present invention the lens holder is connectable to the outer lens of the goggles suitable for winter sports. The 3D lens holder 100 becomes the inner lens 210 of the goggles. The region 120 of the lens holder consists, in the example of FIG. 1, of one element with two halves for the mounting or positioning of two optical lenses 130,140. The lens holder can alternatively also exist out of two independent parts that can be fixed together by a connector, for example a semi flexible or rigid connection part. The first half contains a first opening 150 in which a first optical lens can be mounted or positioned. In embodiments of the present invention the orientation of the optical axis of the optical lens coincides with the optical axis of the eye when the 3D lens holder is mounted in the goggles and the goggles are worn, i.e. put on the head of the user. The maximum deviation of the angle of the axis of the optical lens and the optical axis of the eye will not exceed 10°, preferably will not exceed 5°. In some cases the axis of the optical lens and the optical axis of the eye coincide. In between the second part of the first half and the second part of the second half nasal space is left. This way the optical lenses 130,140 can be positioned closer to the eyes.

In the example of FIG. 2 a 3D view of the lens holder functioning as an inner lens 210 is shown in accordance with an embodiment of the present invention. The edges of the lens holder are connectable to the edges of the outer lens of the goggles suitable for winter sports, e.g. ski goggles.

In some embodiments of the present invention, a groove 810 has been cut in the outer perimeter of the optical lenses 130, 140. In the lens holder, openings 150, 160 have been made in a way that the optical lenses can be clamped. When the optical lenses are clamped in the lens holder, the edge of the openings is positioned in the groove 810 in the perimeter of the optical lens. In some embodiments of the present invention this groove has a depth of 0,1 to 3 mm for instance 0,8 mm, although embodiments are not limited thereby. By deepening the groove, the overlap between the lens holder and the optical lens can be increased and the mechanical fixation can be made stronger. The width of the groove may vary between 0.1 mm and 3 mm. Depending on the thickness of the optical lenses, the width can be selected smaller or wider.

In some embodiments of the present invention a silicon rubber can be inserted in between the lens holder 100 and the optical lens to strengthen the mechanical fixation.

In some embodiments of the present invention the lens holder 100 can be made stiffer to strengthen the mechanical connection between the optical lenses 130,140 and the lens holder. The used materials can be the same as or can be different from the material used for the lens holder.

In some embodiments of the present invention the lens holder 100 and the optical lenses can be produced by 3D printing techniques. In some case the lens holder and the optical lenses can be printed as one and the same 3D print.

In some embodiments of the present invention the surface of the optical lenses can be reduced to decrease the forces acting on it.

In a second aspect the present invention concerns goggles suitable for winter sports, such as for example ski goggles 300, whereby the goggles 300 contain a lens holder 100 as described in the first aspect. In some embodiments of the present invention, the goggles suitable for winter sports comprise optical lenses 130, 140 mounted or positioned in the lens holder. The external edge 110 of the 3D lens holder is mounted or positioned against the outer lens of the goggles.

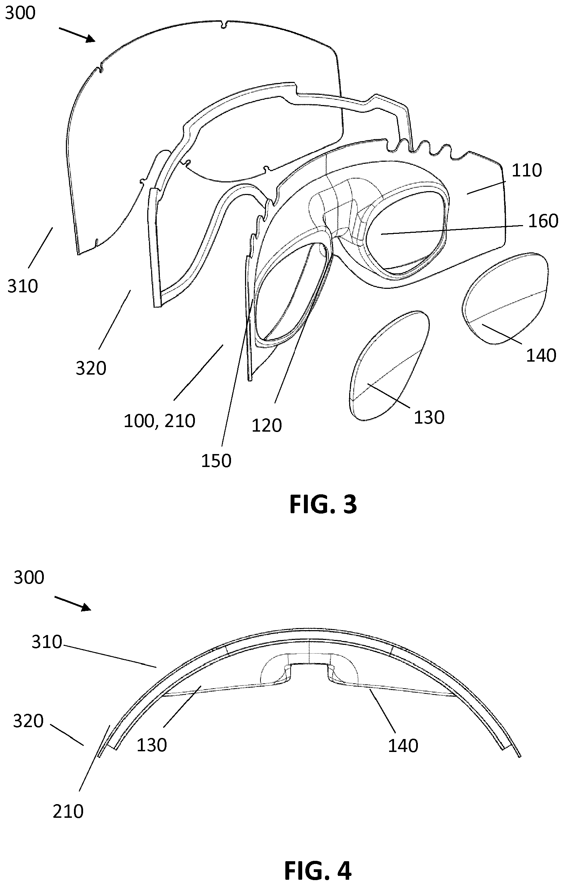

The example of FIG. 3 shows an exploded view of ski goggles 300 with a 3D lens holder and an external lens or visor 310. Between the outer edge of the lens holder and the external lens 310 a foam divider 320 is used to mount the lens holder to the external lens in a closely manner, e.g. to seal the volume between the outer and the inner lens (in this case the 3D lens holder with optical lenses). A typical example of the foam divider is a layer of polyurethane, although the invention is not limited thereby. Other examples, also not limiting embodiments of the present invention, are polyoxymethylene, polyacrylate, expandable polypropylene, or ethylene vinyl acetate. In order to allow easy mounting, this foam layer may be a double-sided adhesive foam layer. In embodiments of the present invention, this foam layer has a width between 3 mm and 15 mm and a thickness, measured from the inner lens to the outer lens, comprised between 1 mm and 15 mm. The space is thus closed by the 3D lens holder, the optical lenses 130, 140 and the external lens.

In the example of FIG. 4, a top view of the same embodiment as shown in the example of FIG. 3, is provided. In this case the 3D lens holder and the outer lens have been sealed together by a foam layer.

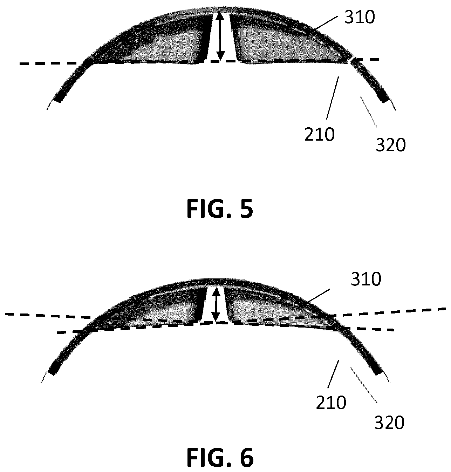

In FIG. 5 the design is shown of a lens holder 100 in accordance with embodiments of the present invention. The figure shows the top view of a pair of ski goggles 300. The lens holder 100 is mounted with the outer edge 110 against the inner lens 210. The goggles 300 have a double lens consisting of an inner lens 210 and outer lens 310. The optical lenses 130, 140 are not visible in the figure, but are positioned or mounted in the region 120 of the lens holder 100. This region 120 of the lens holder consists of two halves. Each half consists of a first part in which an optical lens can be positioned or mounted and a second part which forms the connection through the outer edge between the lens holder and the outer lens of the goggles. The region in which the lenses are mounted is, in the example of FIG. 5, located or positioned in the same plane. This makes it possible to mount or position the optical lenses in one plane.

In some embodiments of the present invention, the axes perpendicular to the first part of the first half and the first part of the second half form an angle between 2x2 ° and 2×8 °, for example between 2×4 ° and 2×6 °, for example, 2×5 °. In the example of FIG. 6, the angle is 2×5 ° (the first part of the first half is rotated about 5 ° as well as the first part of the second half). The region 120 is in the example of FIG.6 placed 4 mm closer to the external lens compared to the example in FIG.5 to enable a better airflow. Because of this, after all, there is more space for ventilation behind the rear side of the lens holder 100. In embodiments of the present invention, there are ventilation holes on the top and bottom of the goggles. These vents allow the supply of cold air and the discharge of hot air as needed. For example, the temperature difference between the air inside the goggles and the outside air is kept low. By positioning the region 120 of the lens holder between 3 mm and 8 mm, for example 4 mm closer to the front, and optionally by providing an angle between the optical axes of the lens holders, more space is created for the air flow. Depending on the optical criteria, the optical lenses 130, 140 can more or less be moved forward and the angle between the optical axes of the optical lenses may be larger or smaller. This angle is typically determined by the rotation of the rear side of the lens holder at the height of the first opening 150 and second opening 160.

The difference between the embodiment in FIG. 5 and that in FIG. 6 is a different orientation of the optical lenses and a modified distance between the lenses in the lens holder and the outer lens.

In some embodiments of the present invention, there is a coating applied to the optical lenses 130, 140. This coating may be, for example, arranged only at the inner side of the optical lenses. Such coating prevents condensation onto the lens. For this purpose, for example, surfactants that reduce the surface tension of the water may be used. It is an advantage of embodiments of the present invention that the visibility is not deteriorated by the application of the coating.

The various aspects can be easily combined with each other, and the combinations thus also correspond to embodiments of the present invention.

## Claims

1. A lens holder (100) for mounting or positioning at least one optical lens (130,140) for correcting visual impairment of a user in goggles suitable for winter sports
wherein
- the lens holder (100) comprises an outer edge adapted to be mounted in a closely fitting manner to an outer lens (310) of the goggles suitable for winter sports, and
- the lens holder (100) being an inner lens (210) of the goggles and being adapted for mounting or positioning at least one optical lens (130,140 ) such that, when the lens holder has been mounted in a closely fitting manner by sealing attachment to the outer lens of the goggles, said at least one optical lens is located outside of the minimal surface of the lens holder and such that at least one closed space is defined by the lens holder, said at least one optical lens (130,140) and the outer lens of the goggles adapted for winter sports,
wherein the at least one optical lens comprises a first optical lens and a second optical lens, and wherein the optical axes of the first optical lens and the second optical lens are intersecting at the side of the eyes at an angle smaller than 10°.

2. A lens holder (100) according to claim 1, wherein for said at least one optical lens (130,140), when the lens holder is mounted in a closely fitting manner in the goggles for winter sports and when worn by the user, a vertex distance between the eyes of the user and the optical lens is obtained between 0 and 20 mm.

3. A lens holder (100) according to any one of the preceding claims, wherein the angle between the optical axis of the first lens and the optical axis of the second lens is smaller than 5°.

4. A lens holder (100) according to any one of the preceding claims, wherein the at least one optical lens (130, 140) can be positioned fixed in at least one opening (150, 160) of the lens holder (100).

5. A lens holder (100) according to claim 4, wherein the at least one optical lens (130, 140) has a groove (810), and wherein the edge of the at least one opening (150.160) of the lens holder (100) is adapted to be clamped in the groove of the at least one optical lens (130, 140).

6. A lens holder (100) according to any one of claims 4 or 5, wherein the at least one optical lens (130, 140) can be mechanically fixed in the at least one opening of the lens holder (100), and wherein the mechanical fixation can be improved by means of a silicone rubber or glue or a combination of both.

7. A lens holder (100) according to any one of the preceding claims, wherein the lens holder and/or one or a plurality of lenses positioned in the lens holder, are provided with an anti-condensation coating.

8. A pair of goggles suitable for winter sports (300), the pair of goggles suitable for winter sports (300) comprising a lens holder (100) according to any one of the preceding claims.

9. A pair of goggles according to claim 8, wherein the lens holder (100) is mounted in a closely fitting manner to the outer lens of the pair of goggles, such that the lens holder (100), the at least one optical lens positioned or provided in the lens holder, and the outer lens of the pair of goggles form a closed space.

10. A pair of goggles (300) according to any one of claims 8 or 9, wherein a surface of the pair of goggles comprises an anti-fog coating.

## Patentansprüche

1. Ein Linsenhalter (100) zum Anbringen oder Positionieren mindestens einer optischen Linse (130, 140) zum Korrigieren einer Sehschwäche eines Benutzers in einer für Wintersport geeigneten Brille, wobei
- der Linsenhalter (100) einen äußeren Rand umfasst, der dazu geeignet ist, eng anliegend an einer äußeren Linse (310) der für Wintersport geeigneten Brille angebracht zu werden, und
- der Linsenhalter (100) eine innere Linse (210) der Brille ist und dazu geeignet ist, mindestens eine optische Linse (130, 140) so anzubringen oder zu positionieren, dass, wenn der Linsenhalter eng anliegend durch dichtende Befestigung an der äußeren Linse der Brille angebracht worden ist, sich die mindestens eine optische Linse außerhalb der minimalen Oberfläche des Linsenhalters befindet und so, dass mindestens ein geschlossener Raum durch den Linsenhalter, die mindestens eine optische Linse (130, 140) und die äußere Linse der für Wintersport geeigneten Brille definiert wird,
wobei die mindestens eine optische Linse eine erste optische Linse und eine zweite optische Linse umfasst, und wobei die optischen Achsen der ersten optischen Linse und der zweiten optischen Linse sich auf der Seite der Augen in einem Winkel von weniger als 10° kreuzen.

2. Ein Linsenhalter (100) nach Anspruch 1, wobei für die mindestens eine optische Linse (130, 140), wenn der Linsenhalter eng anliegend in der für Wintersport geeigneten Brille angebracht ist und vom Benutzer getragen wird, ein Scheitelpunktabstand zwischen den Augen des Benutzers und der optischen Linse zwischen 0 und 20 mm erhalten wird.

3. Ein Linsenhalter (100) nach einem der vorstehenden Ansprüche, wobei der Winkel zwischen der optischen Achse der ersten Linse und der optischen Achse der zweiten Linse kleiner als 5° ist.

4. Ein Linsenhalter (100) nach einem der vorstehenden Ansprüche, wobei die mindestens eine optische Linse (130, 140) fest in mindestens einer Öffnung (150, 160) des Linsenhalters (100) positioniert werden kann.

5. Ein Linsenhalter (100) nach Anspruch 4, wobei die mindestens eine optische Linse (130, 140) eine Nut (810) aufweist, und wobei der Rand der mindestens einen Öffnung (150, 160) des Linsenhalters (100) dazu geeignet ist, in die Nut der mindestens einen optischen Linse (130, 140) geklemmt zu werden.

6. Ein Linsenhalter (100) nach einem der Ansprüche 4 oder 5, wobei die mindestens eine optische Linse (130, 140) mechanisch in der mindestens einen Öffnung des Linsenhalters (100) fixiert werden kann, und wobei die mechanische Fixierung mittels eines Silikonkautschuks oder Klebstoffs oder einer Kombination aus beiden verbessert werden kann.

7. Ein Linsenhalter (100) nach einem der vorstehenden Ansprüche, wobei der Linsenhalter und/oder eine Vielzahl von Linsen, die in dem Linsenhalter positioniert sind, mit einer Antikondensationsbeschichtung versehen sind.

8. Eine für Wintersport geeignete Brille (300), wobei die für Wintersport geeignete Brille (300) einen Linsenhalter (100) nach einem der vorstehenden Ansprüche umfasst.

9. Eine Brille nach Anspruch 8, wobei der Linsenhalter (100) eng anliegend an der äußeren Linse der Brille angebracht ist, sodass der Linsenhalter (100), die mindestens eine optische Linse, die in dem Linsenhalter positioniert oder vorgesehen ist, und die äußere Linse der Brille einen geschlossenen Raum bilden.

10. Eine Brille (300) nach einem der Ansprüche 8 oder 9, wobei eine Oberfläche der Brille eine Antibeschlagbeschichtung umfasst.

## Revendications

1. Un porte-lentille (100) pour monter ou positionner au moins une lentille optique (130,140) pour corriger le défaut visuel d'un utilisateur dans des lunettes adaptées aux sports d'hiver, dans lequel :
- le porte-lentille (100) comprend un bord extérieur adapté pour être monté de manière étroitement ajustée à une lentille extérieure (310) des lunettes adaptées aux sports d'hiver, et
- le porte-lentille (100) étant une lentille intérieure (210) des lunettes et étant adapté pour monter ou positionner au moins une lentille optique (130,140) de telle sorte que, lorsque le porte-lentille a été monté de manière étroitement ajustée par fixation étanche à la lentille extérieure des lunettes, ladite au moins une lentille optique est située à l'extérieur de la surface minimale du porte-lentille et de telle sorte qu'au moins un espace fermé est défini par le porte-lentille, ladite au moins une lentille optique (130,140) et la lentille extérieure des lunettes adaptées pour les sports d'hiver,
dans lequel la ou les lentilles optiques comprennent une première lentille optique et une seconde lentille optique, et dans lequel les axes optiques de la première lentille optique et de la seconde lentille optique se croisent du côté des yeux à un angle inférieur à 10°.

2. Un porte-lentille (100) selon la revendication 1, dans lequel pour ladite au moins une lentille optique (130,140), lorsque le porte-lentille est monté de manière étroitement ajustée dans les lunettes pour sports d'hiver et porté par l'utilisateur, une distance vertex entre les yeux de l'utilisateur et la lentille optique est obtenue entre 0 et 20 mm.

3. Un porte-lentille (100) selon l'une quelconque des revendications précédentes, dans lequel l'angle entre l'axe optique de la première lentille et l'axe optique de la seconde lentille est inférieur à 5°.

4. Un porte-lentille (100) selon l'une quelconque des revendications précédentes, dans lequel la ou les lentilles optiques (130, 140) peuvent être positionnées de manière fixe dans au moins une ouverture (150, 160) du porte-lentille (100).

5. Un porte-lentille (100) selon la revendication 4, dans lequel la ou les lentilles optiques (130, 140) ont une rainure (810), et dans lequel le bord de la ou des ouvertures (150,160) du porte-lentille (100) est adapté pour être serré dans la rainure de la ou des lentilles optiques (130, 140).

6. Un porte-lentille (100) selon l'une quelconque des revendications 4 ou 5, dans lequel la ou les lentilles optiques (130, 140) peuvent être fixées mécaniquement dans la ou les ouvertures du porte-lentille (100), et dans lequel la fixation mécanique peut être améliorée au moyen de caoutchouc silicone ou de colle ou d'une combinaison des deux.

7. Un porte-lentille (100) selon l'une quelconque des revendications précédentes, dans lequel le porte-lentille et/ou une ou plusieurs lentilles positionnées dans le porte-lentille, sont pourvus d'un revêtement anti-condensation.

8. Une paire de lunettes adaptées aux sports d'hiver (300), la paire de lunettes adaptées aux sports d'hiver (300) comprenant un porte-lentille (100) selon l'une quelconque des revendications précédentes.

9. Une paire de lunettes selon la revendication 8, dans laquelle le porte-lentille (100) est monté de manière étroitement ajustée à la lentille extérieure de la paire de lunettes, de sorte que le porte-lentille (100), la ou les lentilles optiques positionnées ou fournies dans le porte-lentille, et la lentille extérieure de la paire de lunettes forment un espace fermé.

10. Une paire de lunettes (300) selon l'une quelconque des revendications 8 ou 9, dans laquelle une surface de la paire de lunettes comprend un revêtement anti-buée.
